# EUROPEAN PATENT APPLICATION

(11) **EP 1 704 861 A1**
(43) Date of publication of application: **27.09.2006**
(21) Application number: 04807717.6
(22) Date of filing: 24.12.2004
(51) Int. Cl.: A61K 31/23, A61K 31/231, A61P 3/04, A61P 3/06, A61P 43/00, A23L 1/30, C12N 9/99

(54) **LIPASE INHIBITOR**

(30) Priority: 16.01.2004 JP 2004008643; 26.11.2004 JP 2004341343; 30.11.2004 JP 2004345099; 20.12.2004 JP 2004366955
(71) Applicant: Fuji Oil Company, Ltd., Osaka-shi, Osaka 542-0086 (JP)
(72) Inventor: ARISHIMA, Toshiharu c/o Fuji Oil Company, Ltd., Tsukuba-gun, Ibaraki 3002436 (JP); TACHIBANA, Nobuhiko c/o Fuji Oil Company, Ltd., Izumisano-shi, Osaka 5988540 (JP)
(74) Representative: Hayes, Adrian Chetwynd
(86) International application number: PCT/JP2004/019360
(87) International publication number: WO 2005/067913

(57) **Abstract**

An object of the present invention is to provide a fat-soluble lipase inhibitor which can contribute to prevention or treatment of obesity due to excessive fat intake or diseases caused by obesity, can be added to fats and oils of all types, and can mildly inhibit hydrolysis by lipase. The present invention is a lipase inhibitor containing, as the active ingredient, at least one substance which is a fat-soluble substance selected from among SLS type triacylglycerols (i.e., symmetric triacylglycerols composed of S which represents a short-chain fatty acid having from 2 to 6 carbon atoms and L which represents a long-chain fatty acid having from 16 to 22 carbon atoms), LUU type and UUL type triacylglycerols (i.e., asymmetric triacylglycerols composed of L which represents a long-chain saturated fatty acid having from 16 to 22 carbon atoms and U which represents an unsaturated fatty acid having from 16 to 22 carbon atoms) and glyceryl ether lipids wherein a long-chain alkyl or alkenyl chain is attached to the 1- or 3-position of the glycerin via an ether bond.

## Description

### Technical Field

The present invention relates to a lipase inhibitor and a food product containing the same. More particularly, the present invention relates to a highly safe fat-soluble lipase inhibitor that can effectively inhibit pancreatic lipase, which is responsible for digestion and absorption of lipid in a living body and the key to obesity and hyperlipemia, and thereby can contribute to suppression or prevention of these diseases.

### Background Art

In recent years, a relationship between obesity due to excessive intake of fat from meals and lifestyle diseases including diabetes, hyperlipemia and circulatory disease has attracted attention and been regarded as a problem. As a method of solving this problem, (1) reducing the fat content of a food product, (2) reducing the calorie content of a fat itself, (3) replacing a fat with a fat substitute, (4) promoting metabolism, (5) using a lipase inhibitor, and the like have been proposed.

Although reducing the fat content of a food product is the shortest way to reduced calorie intake, it deteriorates the texture, taste and physical property of the food product and thereby use of this method is restricted. For the purpose of reducing the calorie content of a fat itself, various structured lipids have been studied. A precondition for reducing calorie intake by using a structured lipid is ingestion of a large amount of a structured lipid. The structure of a structured lipid is specified and therefore its physical property is uniformed. Thus, in actual use of structured lipids for manufacture of food products, structured lipids are not versatile because of their texture, tastes, physical property and the like. For searching fat substitutes, sucrose polyester, finely-divided protein and the like have been studied. However, a product having such taste and function as can substitute for fats has not yet been obtained. Further, for the purpose of preventing ingested fats from accumulating in the body, a substance capable of promoting lipid metabolism, in particular fat burning, has been studied. However, it is difficult to say that such a substance has a clear effect on excessive fat intake and it is also thought that such a substance may be a burden on a living body.

With regard to lipase inhibitors, development of a drug for suppressing or preventing obesity due to excessive lipid intake by partially inhibiting degradation of ingested lipids with pancreatic lipase in a duodenum to reduce digestion and absorption of the lipids has been tried in recent years. According to this concept, the kinds and amounts of fats contained in a food product are not particularly restricted, so that it is expected that such a drug can reduce calorie intake without deteriorating the taste of an existing food product. For example, JP-A 2002-179586 proposes a lipase inhibitor containing at least one selected from the plant group consisting of Rhodiola sacra, roseroot (Rhodiola rosea), soapwort (Saponalia officinalis), boldo (Peumus boldus), Granium dielsianum Knuth, Potentilla tormentilla Scbrank, hercampuri (Gentianella alborocea), Limonium wrigbitii O. Kuntze, chuchuhuasi (Maytenus laevis), cat's claw (Uncaria tomeotosa), cinnamon (Cinnamomum zeylanicum), Zanthoxylum piperitum, Bidens biternate, Acanthopanax sieboldianus, strawberry (Fragaria ananassa), Schinus molle, rose (Rosa hybrida), persimmon (Diospyros kaki Thunb.), St. John's wort (Hypericum perforatum), Chinese gutta-percha (Eucommia ulmoides) and white tea.

JP-A 2002-275077 proposes a lipase inhibitor containing as an active ingredient an extract from at least one material selected from the group consisting of yucca, Panax Ginseng, jasmine tea, hawthorn, Kohki Tea, rooibos tea, soybean germ, ginger and Chinese gutta-percha.

However, almost all of these extracts are watersoluble, so that they can not be mixed into a fat and have to be given when eating or drinking, which is troublesome. In addition, the effect of such extracts may be insufficient. Thus, almost all of them have not come on the market.

Regarding a fat-soluble substance capable of dissolving in a fat, USP 4598089 proposes use of tetrahydrolipstatin as a gastrointestinal lipase inhibitor. The inhibitor is said to directly covalently bind with lipase itself to inactivate it. The effect of the inhibitor is considerably strong and in some cases, results in diarrheal symptom. Thus, there remains concern about safety when the inhibitor is used for food. Therefore, there is a need for a fat-soluble lipase inhibitor that exerts the effect more mildly.

### Disclosure of Invention

### Problem to be solved by the invention:

An object of the present invention is to provide a fat-soluble lipase inhibitor which can contribute to prevention or treatment of obesity due to excessive fat intake or diseases caused by obesity, can be added to fats and oils of all types, and can mildly inhibit hydrolysis by lipase.

### Means for Solving the Problem:

The present inventors intensively studied in order to solve the aforementioned problem, and as a result, found that the degradation rate of the whole fat ingredient could be reduced by incorporating a small amount of at least one substance which is a fat-soluble substance selected from among SLS type triacylglycerols (i.e. symmetric triacylglycerols composed of S which represents a short-chain fatty acid having from 2 to 6 carbon atoms and L which represents a long-chain fatty acid having from 16 to 22 carbon atoms), LUU type and UUL type triacylglycerols (i.e., asymmetric triacylglycerols composed of L which represents a long-chain saturated fatty acid having from 16 to 22 carbon atoms and U which represents an unsaturated fatty acid having from 16 to 22 carbon atoms) and glyceryl ether lipids wherein a long-chain alkyl chain or an alkenyl chain is attached to the 1- or 3- position of glycerin via an ether bond into a base fat.

That is, the first aspect of the present invention is a lipase inhibitor containing, as the active ingredient, at least one substance which is a fat-soluble substance selected from among SLS type triacylglycerols (i.e. symmetric triacylglycerols composed of S which represents a short-chain fatty acid having from 2 to 6 carbon atoms and L which represents a long-chain fatty acid having from 16 to 22 carbon atoms), LUU type and UUL type triacylglycerols (i.e., asymmetric triacylglycerols composed of L which represents a long-chain saturated fatty acid having from 16 to 22 carbon atoms and U which represents an unsaturated fatty acid having from 16 to 22 carbon atoms) and glyceryl ether lipids wherein a long-chain alkyl or alkenyl chain is attached to the 1- or 3- position of the glycerin via an ether bond. The second aspect of the present invention is the lipase inhibitor according to the first aspect wherein the selected fat-soluble substance as the active ingredient is an SLS type triacylglycerol (i.e., symmetric triacylglycerol composed of S which represents a short-chain fatty acid having from 2 to 6 carbon atoms and L represents a long-chain fatty acid having from 16 to 22 carbon atoms). The third aspect of the present invention is a lipid absorption inhibitor containing the SLS type triacylglycerol described in the second aspect as the active ingredient. The forth aspect of the present invention is an anti-obesity agent containing the SLS type triacylglycerol described in the second aspect as the active ingredient. The fifth aspect of the present invention is a hyperlipemia ameliorating agent containing the SLS type triacylglycerol described in the second aspect as the active ingredient. The sixth aspect of the present invention is a food product containing the agent according to any one of the 2nd to 5th aspects. The seventh aspect of the present invention is a pharmaceutical composition containing the agent according to any one of the 2nd to 5th aspects. The eighth aspect of the present invention is the lipase inhibitor according to the first aspect wherein the selected fat-soluble substance as the active ingredient is LUU type and UUL type triacylglycerols (i.e., asymmetric triacylglycerols composed of L which represents a long-chain saturated fatty acid having from 16 to 22 carbon atoms and U which represents an unsaturated fatty acid having from 16 to 22 carbon atoms). The ninth aspect of the present invention is a lipid absorption inhibitor containing the asymmetric triacylglycerol described in the eighth aspect as the active ingredient. The tenth aspect of the present invention is an anti-obesity agent containing the asymmetric triacylglycerol described in the eighth aspect as the active ingredient. The eleventh aspect of the present invention is a hyperlipemia ameliorating agent containing the asymmetric triacylglycerol described in the eighth aspect as the active ingredient. The twelfth aspect of the present invention is a food product containing the agent according to any one of the 8th to 11th aspects. The thirteenth aspect of the present invention is a pharmaceutical composition containing the agent according to any one of the 8th to 11th aspects. The fourteenth aspect of the present invention is the lipase inhibitor according to the first aspect wherein the selected fat-soluble substance as the active ingredient is a glyceryl ether lipid in which a long-chain alkyl or alkenyl chain is attached to the 1-position or the 3- position of the glycerin via an ether bond. The fifteenth aspect of the present invention is a lipid absorption inhibitor containing the glyceryl ether lipid described in the fourteenth aspect as the active ingredient. The sixteenth aspect of the present invention is an anti-obesity agent containing the glyceryl ether lipid described in the fourteenth aspect as the active ingredient. The seventeenth aspect of the present invention is a hyperlipemia ameliorating agent containing the glyceryl ether lipid described in the fourteenth aspect as the active ingredient. The eighteenth aspect of the present invention is a food product containing the agent according to any one of the 14th to 17th aspects. The nineteenth aspect of the present invention is a pharmaceutical composition containing the agent according to any one of the 14th to 17th aspects.

### Effect of the Invention:

The at least one substance selected from among SLS type triacylglycerols (i.e. symmetric triacylglycerols composed of S which represents a short-chain fatty acid having from 2 to 6 carbon atoms and L which represents a long-chain fatty acid having from 16 to 22 carbon atoms), LUU type and UUL type triacylglycerols (i.e., asymmetric triacylglycerols composed of L which represents a long-chain saturated fatty acid having from 16 to 22 carbon atoms and U which represents an unsaturated fatty acid having from 16 to 22 carbon atoms) and glyceryl ether lipids wherein a long-chain alkyl chain or an alkenyl chain is attached to the 1- or 3- position of glycerin via an ether bond, of the present invention can inhibit lipase activity mildly and can be added to fats and oils of all types because it is fat-soluble, so that it is effective in preventing or treating obesity due to excessive fat intake or diseases caused by obesity.

### Best Mode for Carrying Out the Invention

### (First embodiment)

For the first embodiment, in the SLS type triacylglycerol which is a fat-soluble substance capable of delaying lipase hydrolysis, a fatty acid bound to the 1,3-positions of the glycerol is a fatty acid having 2 to 6 carbon atoms including from acetic acid to caproic acid, preferably acetic acid having 2 carbon atoms, and a fatty acid bound to the 2-position of the glycerol is a fatty acid having 16 to 22 carbon atoms including from palmitic acid and palmitoleic acid to behenic acid and erucic acid. The long-chain fatty acid may be a saturated acid or an unsaturated acid, and preferably a mono-unsaturated fatty acid. Examples of the SLS type triacylglycerol include 2P2 (triacylglycerol in which acetic acid is at the 1,3-positions and palmitic acid is at the 2-position), 202 (triacylglycerol in which acetic acid is at the 1,3-positions and oleic acid is at the 2-position), 2Li2 (triacylglycerol in which acetic acid is at the 1,3-positions and linoleic acid is at the 2-position), 2S2 (triacylglycerol in which acetic acid is at the 1,3-positions and stearic acid is at the 2-position), 2A2 (triacylglycerol in which acetic acid is at the 1,3-positions and arachidic acid is at the 2-position), 2B2 (triacylglycerol in which acetic acid is at the 1,3-positions and behenic acid is at the 2-position), 2E2 (triacylglycerol in which acetic acid is at the 1,3-positions and erucic acid is at the 2-position), 4P4 (triacylglycerol in which butyric acid is at the 1,3-positions and palmitic acid is at the 2-position), 404 (triacylglycerol in which butyric acid is at the 1,3-positions and oleic acid is at the 2-position), 4S4 (triacylglycerol in which butyric acid is at the 1,3-positions and stearic acid is at the 2-position), 4A4 (triacylglycerol in which butyric acid is at the 1,3-positions and arachidic acid is at the 2-position), 4B4 (triacylglycerol in which butyric acid is at the 1,3-positions and behenic acid is at the 2-position), 4E4 (triacylglycerol in which butyric acid is at the 1,3-positions and erucic acid is at the 2-position), 6P6 (triacylglycerol in which caproic acid is at the 1,3-positions and palmitic acid is at the 2-position), 6O6 (triacylglycerol in which caproic acid is at the 1,3-positions and oleic acid is at the 2-position), 6S6 (triacylglycerol in which caproic acid is at the 1,3-positions and stearic acid is at the 2-position), 6A6 (triacylglycerol in which caproic acid is at the 1,3-positions and arachidic acid is at the 2-position), 6B6 (triacylglycerol in which caproic acid is at the 1,3-positions and behenic acid is at the 2-position), 6E6 (triacylglycerol in which caproic acid is at the 1,3-positions and erucic acid is at the 2-position), 2P4 (triacylglycerol in which acetic acid is at the 1-position, palmitic acid is at the 2-position, and butyric acid is at the 3-position), 206 (triacylglycerol in which butyric acid is at the 1-position, oleic acid is at the 2-position, and caproic acid is at the 3-position), 4S6 (triacylglycerol in which butyric acid is at the 1-position, stearic acid is at the 2-position, and caproic acid is at the 3-position) and the like. Among them, 202 (triacylglycerol in which acetic acid is at the 1,3-positions and oleic acid is at the 2-position) is preferable.

The SLS type fat is hard to hydrolyze by lipase because it has short-chain fatty acids at the 1,3-positions. Thus, lipase hydrolysis of a fat can be reduced or delayed by incorporating 0.5 to 35% by weight, preferably 0.5 to 20% by weight, more preferably 1 to 10% by weight of the SLS type triacylglycerol into the fat.

The degree of reduction or delay in hydrolysis of a fat by a lipase inhibitor should be preferably mild, that is, a mild inhibiting effect is preferable. Ideally, the lipase inhibitor induces difficulty in degradation of about 10 to 30%, preferably about 10 to 20% of a fat ingested.

When the amount of the SLS type triacylglycerol contained in a fat is less than the lower limit, it is difficult to obtain the expected effect. When the amount of the SLS type triacylglycerol contained in a fat exceeds the upper limit, the taste is deteriorated and the effect becomes too strong.

The SLS type triacylglycerol of the present invention is generally obtained by mixing a fat derived from an animal, a plant or a fish with a short-chain fatty acid or a lower alcohol ester thereof such as ethyl ester in appropriate proportion, and then subjecting the mixture to transesterification using a 1,3-position specific lipase by a known method. Examples of a fat derived from a plant include soybean oil, rapeseed oil, palm oil, cottonseed oil, sunflower oil, corn oil, canola oil and the like. Examples of a fat derived from an animal include beef tallow, lard, fish oil and the like. Alternatively, the SLS type triacylglycerol may be prepared by non-selective transesterification, for example, using an alkali catalyst such as sodium methylate. In this case, however, a positional isomer SSL type triacylglycerol is produced twice the production amount of the SLS type triacylglycerol, so that the effect decreases, which is not practicable. The SLS type triacylglycerol thus obtained can be then subjected to distillation, fractionation, and if necessary, conventional processing treatment such as hardening, to obtain a product having a purity of 70% or more.

The SLS type triacylglcerol of the present invention may be used as it is or as a mixture with other fats. The mixing ratio varies depending on the expected effect and a system to be used, and a fat to be mixed is not limited as long as it is an edible fat derived form an animal or a plant.

The SLS type triacylglycerol of the present invention can be widely incorporated into food products usually containing fats. For example, the SLS type triacylglycerol of the present invention can be added to emulsified food products including cream, margarine, mayonnaise, dressing and dairy products, confectionaries including chocolate, breads, processed meat products including ham and sausage, processed marine products including steamed fish paste (kamaboko) and a tubular roll of grilled fish paste (chikuwa), and the like. When the SLS type triacylglycerol of the present invention is incorporated into these food products, their taste and texture are not deteriorated. Although short-chain fatty acids which are present in the SLS type traicylglycerol are slightly unstable at a high temperature, the SLS type traicylglycerol can be of course used in cooking or frying. Alternatively, the SLS type traicylglycerol can be added to water, fruit juice, cow milk, tea or soft drink that is ingested simultaneously with the aforementioned food products even if they do not contain fats.

The lipase inhibitor, the lipid absorption inhibitor, the anti-obesity agent, the hyperlipemia ameliorating agent and the pharmaceutical compositions containing them of the present invention may be administered orally or parenterally. For administration, the active ingredient can be mixed with a solid or liquid pharmaceutical carrier suitable for an administration method such as oral administration, rectal administration or injection and then be administered as a formulation.

### Examples

Hereinafter, the present invention will be explained in more detail by reference to the following Examples which the spirit of the present invention is not limited to. In Examples, both of % and part are based on weight.

### Preparation Example 1

50 parts of a high-oleic sunflower oil having an iodine value of 84 and 50 parts of ethyl acetate having a purity of 99.5% were mixed and then subjected to transesterification using a 1,3-position specific lipase (Novozaymes, Lipozyme RM-IM) to obtain a reaction oil. The reaction oil was distilled at 220°C to remove esters and further heated to obtain 10 parts of a 202 fraction having a purity of 83% at 250°C.

### Pharmacological test 1

Soybean oils of which 1 part (202 purity: about 0.8%), 5 parts (202 purity: about 4.2%), 10 parts (202 purity: about 8.3%) and 50 parts (202 purity about: 41.5%) were replaced with the 202 fraction obtained as described above, and the 202 fraction itself (202 purity: 83%) were used for the following lipase activity measurement. To 80 mg of each oil, 80 mg of phosphatidylcholine (Sigma), 5 mg of sodium taurocholate (Wako Pure Chemical Industries, Ltd.), and 9 ml of a 0.1 M TES buffer (PH 7) containing 0.1 M NaCl were added and the mixture was emulsified with an ultrasound oscillator for 1 minute to obtain a substrate. To 300 µl of the substrate, 5 µl (5U) of a pig pancreatic lipase (Sigma) was added and then reacted at 37°C for 1 hour. To the reaction mixture was then added 3 ml of an extraction solvent (a mixture of chloroform/heptane/methanol = 49 parts/49 parts/ 2 parts). The mixture was stirred vigorously and then centrifuged at 2500 rpm for 5 minutes. The upper layer was removed. To the lower layer was added 1 ml of a copper regent (prepared by dissolving 2.98 g of triethanolamine, 2.42 g of copper nitrate and 0.48 g of NaOH in 200 ml of water and then adding 66 g of NaCl). The mixture was stirred for 10 minutes and then centrifuged at 2500 rpm for 10 minutes to take 1.5 ml of the upper layer as a sample. To the sample was added 1.5 ml of a color-producing reagent (prepared by 0.2 g of bathocuproin and 0.1 g of butylhydroxyanisole in 200 ml of chloroform) and an absorbance at OD480 was measured as the quantity of free fatty acid. Table 1 shows the activity relative to a system containing only soybean oil. As shown in Table 1, the activity of the soybean oil of which 1 part was replaced with 202 was inhibited by about 20%, and the activity of the soybean oil of which 5 parts were replaced with 202 was inhibited by about 35%.

### Comparative Preparation Example 1

50 parts of triacetin having a purity of 98% and 50 parts of ethyl oleate having a purity of 98% were mixed and subjected to transesterification using a 1,3-position specific lipase (Novozaymes, Lipozyme RM-IM) to obtain a reaction oil. The reaction oil was distilled at 220°C to remove esters and further heated to obtain 50 parts of a 220 fraction having a purity of 82% at 250°C.

### Comparative Pharmacological Test 1

Soybean oils of which 1 part (220 purity: about 0.8%), 5 parts (220 purity: about 4.1%), 10 parts (220 purity: about 8.2%) and 50 parts (220 purity: about 41%) were replaced with the 220 fraction obtained as described above, and the 220 fraction itself (220 purity: 82%) were used for lipase activity measurement as described above. As shown in Table 1, there was no inhibitory effect on lipase hydrolysis, regardless of the replacement amounts with 220.

### Comparative Preparation Example 2

55 parts of a high-oleic sunflower oil having an iodine value of 84 and 45 parts of triacetin having a purity of 98% were mixed and then subjected to random transesterificaiton using sodium methylate, followed by neutralization and washing with water to obtain about 60 parts of a reaction oil. The reaction oil was distilled at 250°C to obtain 42 parts of a 220/202 mixed fraction having a purity of 87% (220 purity: about 57%; 202 purity: about 30%).

### Comparative Pharmacological Test 2

Soybean oils of which 1 part (220 purity: about 0.6%, 202 purity: about 0.3%), 5 parts (220 purity: about 2.9%, 202 purity: about 1.5%), 10 parts (220 purity: about 5.7%, 202 purity: about 3.0%) and 50 parts (220 purity: about 28.5%, 202 purity: about 15%) were replaced with the 220/202 mixed fraction obtained as described above, and the 220/202 mixed fraction itself (220 purity: 57%, 202 purity: 30%) were used for lipase activity measurement as described above. As shown in Table 1, even though the replacement amount with 22O/2O2 was increased, there was no remarkable inhibitory effect on lipase hydrolysis.

### Preparation Example 2

50 parts of a high-oleic sunflower oil having an iodine value of 84 and 50 parts of ethyl hexanoate having a purity of 98% were mixed and then subjected to transesterification using a 1,3-position specific lipase (Novozaymes, Lipozyme RM-IM) to obtain a reaction oil. The reaction oil was distilled at 220°C to remove esters and further heated to obtain 15 parts of a 606 fraction having a purity of 78% at 258°C.

### Pharmacological Test 2

Soybean oils of which 1 part (606 purity: about 0.8%), 5 parts (606 purity: about 3.9%), 10 parts (606 purity: about 7.8%) and 50 parts (606 purity: about 39%) were replaced with the 606 fraction obtained as described above, and the 606 fraction itself (606 purity: 78%) were used for lipase activity measurement as described above. As shown in Table 1, the activity of the soybean oil of which 1 part was replaced with 606 was inhibited by about 15%, and the activity of the soybean oil of which 10 parts was replaced with 606 was inhibited by about 25%.

**Table 1 Relative activity of test fat to soybean oil**

| Pharmacological test | Fat composition (part) | Relative activity (%) |
|---|---|---|
| Control (Soybean oil) | 100/0 | 100 |
| Pharmacological test 1 (Soybean oil/202) | 99/1 | 78 |
| | 95/5 | 66 |
| | 90/10 | 67 |
| | 50/50 | 48 |
| | 0/100 | 11 |
| Comparatuve pharmacological test 1 (Soybean oil/220) | 99/1 | 101 |
| | 95/5 | 106 |
| | 90/10 | 111 |
| | 50/50 | 115 |
| | 0/100 | 108 |
| Comparatuve pharmacological test 2 (Soybean oil/220/202) | 99/1 | 100 |
| | 95/5 | 89 |
| | 90/10 | 94 |
| | 50/50 | 113 |
| | 0/100 | 114 |
| Pharmacological test 2(Soybean oil/606) | 99/1 | 86 |
| | 95/5 | 91 |
| | 90/10 | 74 |
| | 50/50 | 68 |
| | 0/100 | 30 |

### Digestion and absorption test in mouse

Using the 202 fraction obtained in Preparation Example 1 and mice, a digestion and absorption test was performed for about 2 months. Seven-week-old C57BL/6J mice were pre-reared for 1 week and used for the test. The compounded feed shown in Table 2, which is prepared by partially modifying AIN-93G composition, or soybean oil as a control was fed to the mice for about 2 months. Each group consisted of 6 mice. After reared for about 2 months, a change in the body weight, feed efficiency and body fat percentage were measured. For measuring body fat percentage, an X-ray bone density measuring apparatus for exclusive use in experimental mice, PIXImus2 (GE Medical Systems) was used. As a result of a digestion and adsorption test on mice for 56 days, in a group using the 202 fraction, the body weight was reduced by about 10%, the feed efficiency was reduced by 17%, and the body fat percentage was reduced by about 14%. Therefore, it was suggested that an addition of a small amount of 202 is effective against obesity due to excessive fat intake. These results are summarized in Table 3.

**Table 2 Feed composition (wt%)**

| Composition | Soybean oil group | Group using 202 fraction |
|---|---|---|
| Soybean oil | 10.0 | - 10.0 |
| 202 fraction (Preparation Example 1) | - | 0.5 |
| Casein | 20.0 | 20.0 |
| Sucrose | 10.0 | 10.0 |
| β-corn starch | 36.75 | 36.25 |
| α-corn starch | 13.2 | 13.2 |
| L-Cystine | 0.3 | 0.3 |
| Cellulose powder | 5.0 | 5.0 |
| Min. mix (AIN-93G) | 3.5 | 3.5 |
| Vit. mix (AIN-93G) | 1.0 | 1.0 |
| Choline bitartrate | 0.25 | 0.25 |
| Total weight | 100.00 | 100.00 |

**Table 3 Results of rearing**

| | | Soybean oil group | Group using 202 fraction |
|---|---|---|---|
| Body weight | Before initiation of rearing | 22.9±0.5 | 22.8±0.3 |
| | After rearing for 56 days | 34.0±1.2 | 30.7±1.4 |
| Feed efficiency | After rearing for 56 days | 0.074±0.003 | 0.061±0.002 |
| Body fat percentage | Before initiation of rearing | 20.2±0.9 | 19.1±0.9 |
| | After rearing for 56 days | 40.5±1.5 | 34.8±2.5 |

### (Second embodiment)

For the second embodiment, in the LUU type and UUL type triacylglycerols which are fat-soluble substances capable of delaying lipase hydrolysis, a fatty acid bound to the 1-position or the 3-position of the glycerol is a saturated fatty acid having 16 to 22 carbon atoms including from palmitic acid to behenic acid, preferably a longer-chain fatty acid, and a fatty acid bound to the 2-position and the 3-position (in the case of LUU type) or the 1-position and the 2-position (in the case of UUL type) is a fatty acid having 16 to 22 carbon atoms including from palmitoleic acid to erucic acid. The long-chain unsaturated fatty acid may be a mono-unsaturated fatty acid or a poly-unsaturated fatty acid, and from a viewpoint of oxidation stability upon actual use, a mono-unsaturated fatty acid is preferable. Examples of the LUU type and UUL type triacylglycerols include SOO (in which stearic acid is at the 1-position and oleic acid is at the 2,3-positions), OOS (in which stearic acid is at the 3-position and oleic acid is at the 1,2-positions), SLiLi (in which stearic acid is at the 1-position and linoleic acid is at the 2,3-positions), SLnLn (in which stearic acid is at the 1-position and linolenic acid is at the 2,3-positions), BOO (in which behenic acid is at the 1-position and oleic acid is at the 2,3-positions), BLiO (in which behenic acid is at the 1-position, linoleic acid is at the 2-position, and oleic acid is at the 3-position) and the like. Among them, BOO (in which behenic acid is at the 1-position and oleic acid is at the 2,3-positions) and OOB (in which behenic acid is at the 3-position and oleic acid is at the 1,2-positions) are preferable.

The fats of LUU type and UUL type triacylglycerols are hard to hydrolyze by lipase because they have a long-chain saturated fatty acid at the 1-position or the 3-position. Thus, lipase hydrolysis of the whole fat ingredient can be reduced or delayed by incorporating 0.5 to 30% by weight, preferably 1 to 15% by weight, more preferably 1 to 10% by weight of the LUU type and UUL type triacylglycerols into a base fat.

The degree of reduction or delay in hydrolysis of a fat by a lipase inhibitor should be preferably mild, that is, a mild inhibiting effect is preferable. Ideally, the lipase inhibitor induces difficulty in degradation of about 10 to 30%, preferably about 10 to 20% of a fat ingested.

A fat having two long-chain saturated fatty acids in the molecule, such as LLU type triacylglycerol or LUL type triacylglycerol, is hard to recognize as a substrate for a lipase in a living body because such a fat has a higher melting point and crystallizes at around the body temperature. Thus, the inhibiting effect of such a fat can not be expected.

The LUU type and UUL type triacylglycerols are generally obtained by mixing a fat containing long-chain saturated fatty acids derived from an animal, a plant or a fish or an ester thereof with a fat containing long-chain unsaturated fatty acids in appropriate proportion, and then subjecting the mixture to transesterification using a 1,3-position specific lipase by a known method. Examples of a fat derived from a plant include soybean oil, rapeseed oil, palm oil, cottonseed oil, sunflower oil, corn oil, canola oil and the like. Examples of a fat derived from an animal include beef tallow, lard, fish oil and the like. Alternatively, The LUU type and UUL type triacylglycerols may be prepared by non-selective transesterification, for example, using an alkali catalyst such as sodium methylate. In this case, however, positional isomers ULU, UUU, LLU, LUL and LLL type triacylglycerols are produced, so that subsequent concentration procedure becomes troublesome, which is not practicable. The LUU type and UUL type fats thus obtained can be then subjected to conventional processing treatment such as fractionation to be concentrated to about 80%.

The LUU type and UUL type triacylglycerols of the present invention may be used as they are or optionally may be blended with other base fats. The blending ratio varies depending on the expected effect and a system to be used, and a base fat to be blended is not limited as long as it is an edible fat derived form an animal or a plant.

The LUU type and UUL type triacylglycerols of the present invention can be widely incorporated into food products usually containing fats. For example, the LUU type and UUL type triacylglycerols of the present invention can be added to emulsified food products including cream, margarine, mayonnaise, dressing and dairy products, confectionaries including chocolate, breads, processed meat products including ham and sausage, processed marine products including steamed fish paste (kamaboko) and a tubular roll of grilled fish paste (chikuwa), and the like. When the LUU type and UUL type triacylglycerols of the present invention are incorporated into these food products, their taste and texture are not deteriorated.

The lipase inhibitor, the lipid absorption inhibitor, the anti-obesity agent, the hyperlipemia ameliorating agent and the pharmaceutical compositions containing them of the present invention may be administered orally or parenterally. For administration, the active ingredient can be mixed with a solid or liquid pharmaceutical carrier suitable for an administration method such as oral administration, rectal administration or injection and then be administered as a formulation.

### Preparation Example 3

50 parts of a high-oleic sunflower oil having an iodine value of 84 and 50 parts of ethyl behenate having a purity of 95% were mixed and then subjected to transesterification using a 1,3-position specific lipase (Novozaymes, Lipozyme RM-IM) to obtain a reaction oil. The reaction oil was distilled to remove esters and then 100 parts of hexane was added. Then, crystallization and fractionation at -5°C afforded 20 parts of a LUU/UUL fraction having a purity of 70% and 30 parts of a LUL fraction having a purity of 78%.

### Pharmacological Test 3

Soybean oils of which 1 part (LUU/UUL purity: about 0.7%), 5 parts (LUU/UUL purity: about 3.5%) and 10 parts (LUU/UUL purity: about 7.0%) were replaced with the LUU/UUL fraction obtained as described above, and the LUU/UUL fraction itself (LUU/UUL purity: 70%) were used for the following lipase activity measurement. To 80 mg of each oil, 80 mg of phosphatidylcholine (Sigma), 5 mg of sodium taurocholate (Wako Pure Chemical Industries, Ltd.), and 9 ml of a 0.1 M TES buffer (PH 7) containing 0.1 M NaCl were added and the mixture was emulsified with an ultrasound oscillator for 1 minute to obtain a substrate. To 300 µl of the substrate, 5 µl (5U) of a pig pancreatic lipase (Sigma) was added and then reacted at 37°C for 1 hour. To the reaction mixture was then added 3 ml of an extraction solvent (a mixture of chloroform/heptane/methanol = 49 parts/49 parts/ 2 parts). The mixture was stirred vigorously and then centrifuged at 2500 rpm for 5 minutes. The upper layer was removed. To the lower layer was added 1 ml of a copper regent (prepared by dissolving 2.98 g of triethanolamine, 2.42 g of copper nitrate and 0.48 g of NaOH in 200 ml of water and then adding 66 g of NaCl). The mixture was stirred for 10 minutes and then centrifuged at 2500 rpm for 10 minutes to take 1.5 ml of the upper layer as a sample. To the sample was added 1.5 ml of a color-producing reagent (prepared by 0.2 g of bathocuproin and 0.1 g of butylhydroxyanisole in 200 ml of chloroform) and an absorbance at OD480 was measured as the quantity of free fatty acid. Table 4 shows the activity relative to a system containing only soybean oil. As shown in Table 4, the activity of the soybean oil of which 1 part was replaced with LUU/UUL was inhibited by about 15%, and the activity of the soybean oil of which 5 parts were replaced with LUU/UUL was inhibited by about 25%.

### Comparative Pharmacological Test 3

Soybean oils of which 1 part (LUL purity: about 0.8%), 5 parts (LUL purity: about 3.9%) and 10 parts (LUL purity: about 7.8%) were replaced with the LUL fraction obtained in Preparation Example 3, and the LUL fraction itself (LUL purity: 78%) were used for lipase activity measurement as described above. As shown in Table 4, even though the replacement amount with LUL was increased, there was no remarkable inhibitory effect on lipase hydrolysis. In addition, partial demulsification due to crystallization was observed. Thus, it was found that there was no inhibitory effect on lipase hydrolysis.

### Comparative Preparation Example 3

40 parts of tribehen having an iodine value of 1, 60 parts of ethyl oleate having a purity of 98% and 300 parts of hexane were mixed and then subjected to transesterification using a 1,3-position specific lipase (Novozymes, Lipozyme RM-IM) to obtain a reaction oil. The solvent was removed and esters were removed by distillation. Then, further 100 parts of hexane was added. The mixture was crystallized and fractionated at a predetermined temperature to obtain 15 parts of a LLU/ULL fraction having a purity of 72% and 10 parts of a ULU fraction having a purity of 70%.

### Comparative Pharmacological Test 4

Soybean oils of which 1 part (LLU/ULL purity: about 0.7%), 5 parts (LLU/ULL purity: about 3.6%) and 10 parts (LLU/ULL purity: about 7.2%) were replaced with the LLU/ULL fraction obtained in Comparative Preparation Example 3, and the LLU/ULL fraction (LLU/ULL purity: 72%) were used for lipase activity measurement as described above. As shown in Table 4, when the replacement amount with LLU/ULL was increased, there was slight inhibitory effect on lipase hydrolysis. However, such effect was not comparable to that of LUU/UUL.

### Comparative Pharmacological Test 5

Soybean oils of which 1 part (ULU purity: about 0.7%), 5 parts (ULU purity: about 3.5%) and 10 parts (ULU purity: about 7.0%) were replaced with the ULU fraction obtained in Comparative Preparation Example 3, and the ULU fraction itself (ULU purity: 70%) were used for lipase activity measurement as described above. As shown in Table 4, even though the replacement amount with ULU was increased, there was no remarkable inhibitory effect on lipase hydrolysis.

**Table 4 Relative activity of test fat to soybean oil**

| Pharmacological test | Fat composition (part) | Relative activity (%) |
|---|---|---|
| Control (soybean oil) | 100/0 | 100 |
| Pharmacological test 3 (Soybean/(LUU/UUL)) | 99/1 | 85 |
| | 95/5 | 75 |
| | 90/10 | 71 |
| | 0/100 | 54 |
| Comparative pharmacological test 3 (Soybean oil/LUL) | 99/1 | 95 |
| | 95/5 | 93 |
| | 90/10 | 95 |
| | 0/100 | 92 |
| Comparative pharmacological test 4 (Soybean oil/(LLU/ULL)) | 99/1 | 99 |
| | 95/5 | 95 |
| | 90/10 | 93 |
| | 0/100 | 89 |
| Comparative pharmacological test 5 (Soybean oil/ULU) | 99/1 | 102 |
| | 95/5 | 99 |
| | 90/10 | 105 |
| | 0/100 | 103 |

### Digestion and absorption test in mouse

Using the LUU/UUL fraction obtained in Preparation Example 3 and mice, a digestion and absorption test was performed for about 2 months. Seven-week-old C57BL/6J mice were pre-reared for 1 week and used for the test. The compounded feed shown in Table 5, which is prepared by partially modifying AIN-93G composition, or soybean oil as a control was fed to the mice for about 2 months. Each group consisted of 6 mice. After reared for about 2 months, a change in the body weight, feed efficiency and body fat percentage were measured. For measuring body fat percentage, an X-ray bone density measuring apparatus for exclusive use in experimental mice, PIXImus2 (GE Medical Systems) was used. As a result of a digestion and adsorption test on mice for 56 days, in a group using the SUU/UUS fraction, the body weight was reduced by about 9%, the feed efficiency was reduced by 12%, and the body fat percentage was reduced by about 13%. Therefore, it was suggested that an addition of a small amount of LUU/UUL is effective against obesity due to excessive fat intake. These results are summarized in Table 6.

**Table 5 Feed composition (wt%)**

| Composition | Soybean oil group | Group using LUU/UUL fraction |
|---|---|---|
| Soybean oil | 10.0 | 10.0 |
| LUU/UUL fraction (test fat) | - | 0.5 |
| Casein | 20.0 | 20.0 |
| Sucrose | 10.0 | 10.0 |
| β-corn starch | 36.75 | 36.25 |
| α-corn starch | 13.2 | 13.2 |
| L-Cystine | 0.3 | 0.3 |
| Cellulose powder | 5.0 | 5.0 |
| Min. mix (AIN-93G) | 3.5 | 3.5 |
| Vit. mix (AIN-93G) | 1.0 | 1.0 |
| Choline bitartrate | 0.25 | 0.25 |
| Total weight | 100.00 | 100.00 |

**Table 6 Results of rearing**

| | | Soybean oil group | Group using LUU/UUL fraction |
|---|---|---|---|
| Body weight | Before initiation of rearing | 22.9±0.5 | 22.8±0.3 |
| | After rearing for 56 days | 34.0±1.2 | 30.8±1.4 |
| Feed efficiency | After rearing for 56 days | 0.074±0.003 | 0.065±0.004 |
| Body fat percentage | Before initiation of rearing | 20.2±0.9 | 19.1±1.0 |
| | After rearing for 56 days | 40.5±1.5 | 35.1±1.4 |

### (Third embodiment)

For the third embodiment, in the glyceryl ether lipid, a long-chain alkyl chain or a long-chain alkenyl chain is attached to the 1-position or the 3-position of the glycerol via an ether bond. Although such structure is easy for a lipase to recognize as a substrate, the existence of the ether bond at the 1-position or the 3-position delays lipase hydrolysis. The length of the alkyl or alkenyl chain is not particularly limited, and C14 to C22 alkyl or alkenyl chain is actually used. The 2-position of the glycerol is not particularly limited, and in order to suppress the rise of the melting point, it is preferable that an unsaturated fatty acid is attached to the 2-position via an ester bond. Examples of the glyceryl ether lipid include triglyceryl ether, monoacyl diglyceryl ether, diacyl monoglyceryl ether and the like.

The glyceryl ether lipid is hard to hydrolysis by lipase because it has an ether bond at the 1-position or the 3-position. Thus, lipase hydrolysis of the whole fat ingredient can be reduced or delayed by incorporating 0.5 to 30% by weight, preferably 1 to 15% by weight, more preferably 1 to 10% by weight of the glyceryl ether lipid into a base fat.

The degree of reduction or delay in hydrolysis of a fat by a lipase inhibitor should be preferably mild, that is, a mild inhibiting effect is preferable. Ideally, the lipase inhibitor induces difficulty in degradation of about 10 to 30%, preferably about 10 to 20% of a fat ingested.

Marine animals and shark liver oil contain generally a large amount of the glyceryl ether lipid, and are compressed or extracted with a solvent and then subjected to distillation, fractionation and the like to prepare the glyceryl ether lipid. Examples of marine animals include shrimp, squid, anchovy and the like. Examples of sharks include chimaera and the like, and in addition, bony fishes such as striped mullet are included. The glyceryl ether lipid thus obtained may be subjected to processing treatment such as enzymatic hydrolysis of the ester part or hydrogenation.

The glyceryl ether lipid of the present invention may be used as it is or optionally may be blended with other base fats. The blending ratio varies depending on the expected effect and a system to be used, and a base fat to be blended is not limited as long as it is an edible fat derived form an animal or a plant.

The glyceryl ether lipid of the present invention can be widely incorporated into food products usually containing fats. For example, the glyceryl ether lipid of the present invention can be added to emulsified food products including cream, margarine, mayonnaise, dressing and dairy products, confectionaries including chocolate, breads, processed meat products including ham and sausage, processed marine products including steamed fish paste (kamaboko) and a tubular roll of grilled fish paste (chikuwa), and the like. When the glyceryl ether lipid of the present invention is incorporated into these food products, their taste and texture are not deteriorated. Alternatively, the glyceryl ether lipid can be added to water, fruit juice, cow milk, tea or soft drink that is ingested simultaneously with the aforementioned food products even if they do not contain fats.

The lipase inhibitor, the lipid absorption inhibitor, the anti-obesity agent, the hyperlipemia ameliorating agent and the pharmaceutical compositions containing them of the present invention may be administered orally or parenterally. For administration, the active ingredient can be mixed with a solid or liquid pharmaceutical carrier suitable for an administration method such as oral administration, rectal administration or injection and then be administered as a formulation.

### Preparation Example 4

100 parts of a commercially available deep-sea shark liver oil (product name: deep-sea shark raw liver oil, manufactured by Kabushikigaisya Miyama Kanpo Seiyaku, composition labeling: glyceryl ether lipid 48%, squalene 45%, fatty acid 7%) were distilled at 230°C to remove 45 parts of squalene and 7 parts of fatty acid and obtain 47 parts of a glyceryl ether lipid fraction (composition: triglyceryl ether 41%, monoacyl diglyceryl ether 19%, diacyl monoglyceryl ether 8%, triacylglycerol 32%).

### Pharmacological Test 4

Soybean oils of which 1 part, 5 parts and 10 parts were replaced with the glyceryl ether lipid fraction obtained as described above, and the glyceryl ether lipid fraction itself were used for the following lipase activity measurement. To 80 mg of each oil, 80 mg of phosphatidylcholine (Sigma), 5 mg of sodium taurocholate (Wako Pure Chemical Industries, Ltd.), and 9 ml of a 0.1 M TES buffer (PH 7) containing 0.1 M NaCl were added and the mixture was emulsified with an ultrasound oscillator for 1 minute to obtain a substrate. To 300 µl of the substrate, 5 µl (5U) of a pig pancreatic lipase (Sigma) was added and then reacted at 37°C for 1 hour. To the reaction mixture was then added 3 ml of an extraction solvent (a mixture of chloroform/heptane/methanol = 49 parts/49 parts/ 2 parts). The mixture was stirred vigorously and then centrifuged at 2500 rpm for 5 minutes. The upper layer was removed. To the lower layer was added 1 ml of a copper regent (prepared by dissolving 2.98 g of triethanolamine, 2.42 g of copper nitrate and 0.48 g of NaOH in 200 ml of water and then adding 66 g of NaCl). The mixture was stirred for 10 minutes and then centrifuged at 2500 rpm for 10 minutes to take 1.5 ml of the upper layer as a sample. To the sample was added 1.5 ml of a color-producing reagent (prepared by 0.2 g of bathocuproin and 0.1 g of butylhydroxyanisole in 200 ml of chloroform) and an absorbance at OD480 was measured as the quantity of free fatty acid. Table 7 shows the activity relative to a system containing only soybean oil. As shown in Table 7, the activity of the soybean oil of which 1 part was replaced with the glyceryl ether lipid was inhibited by about 12%, and the activity of the soybean oil of which 5 parts were replaced with the glyceryl ether lipid was inhibited by about 20%.

**Table 7 Relative activity of test fat to soybean oil**

| Pharmacological test | Fat composition (part) | Relative activity (%) |
|---|---|---|
| Control (soybean oil) | 100/0 | 100 |
| Pharmacological test 4 (Soybean oil/glyceryl ether lipid fraction) | 99/1 | 88 |
| | 95/5 | 80 |
| | 90/10 | 72 |
| | 0/100 | 35 |

### Digestion and absorption test in mouse

Using the glyceryl ether lipid fraction obtained in Preparation Example 4 and mice, a digestion and absorption test was performed for about 2 months. Seven-week-old C57BL/6J mice were pre-reared for 1 week and used for the test. The compounded feed shown in Table 8, which is prepared by partially modifying AIN-93G composition, or soybean oil as a control was fed to the mice for about 2 months. Each group consisted of 6 mice. After reared for about 2 months, a change in the body weight, feed efficiency and body fat percentage were measured. For measuring body fat percentage, an X-ray bone density measuring apparatus for exclusive use in experimental mice, PIXImus2 (GE Medical Systems) was used. As a result of a digestion and adsorption test on mice for 56 days, in a group using soybean oil/the glyceryl ether lipid fraction, the body weight and the feed efficiency were reduced by bout 10%, and the body fat percentage was reduced by about 12%. Therefore, it was suggested that an addition of a small amount of the glyceryl ether lipid is effective against obesity due to excessive fat intake. These results are summarized in Table 9.

**Table 8 Feed composition (wt%)**

| Composition | Soybean oil group | Group using glyceryl ether fat fraction |
|---|---|---|
| Soybean oil | 10.0 | 10.0 |
| Glyceryl ether lipid fraction (Test fat) | - | 0.5 |
| Casein | 20.0 | 20.0 |
| Sucrose | 10.0 | 10.0 |
| β-corn starch | 36.75 | 36.25 |
| α-corn starch | 13.2 | 13.2 |
| L-Cystine | 0.3 | 0.3 |
| Cellulose powder | 5.0 | 5.0 |
| Min. mix (AIN-93G) | 3.5 | 3.5 |
| Vit. mix (AIN-93G) | 1.0 | 1.0 |
| Choline bitartrate | 0.25 | 0.25 |
| Total weight | 100.00 | 100.00 |

**Table 9 Results of rearing**

| | | Soybean oil group | Group using glyceryl ether lipid fraction |
|---|---|---|---|
| Body weight | Before initiation of rearing | 22.9±0.5 | 23.3±0.3 |
| | After rearing for 56 days | 34.0±1.2 | 30.7±1.7 |
| Feed efficiency | After rearing for 56 days | 0.074±0.003 | 0.066±0.004 |
| Body fat percentage | Before initiation of rearing | 20.2±0.9 | 19.7±1.0 |
| | After rearing for 56 days | 40.5±1.5 | 35.6±2.5 |

### Industrial Applicability

According to the present invention, it is possible to obtain a lipase inhibitor which mildly inhibits lipase activity, is fat-soluble, can be added to edible fats and oils of all types, and is effective in preventing or treating obesity due to excessive fat intake or diseases caused by obesity; and a fat composition containing the lipase inhibitor.

## Claims

1. A lipase inhibitor containing, as the active ingredient, at least one substance which is a fat-soluble substance selected from among SLS type triacylglycerols (i.e. symmetric triacylglycerols composed of S which represents a short-chain fatty acid having from 2 to 6 carbon atoms and L which represents a long-chain fatty acid having from 16 to 22 carbon atoms), LUU type and UUL type triacylglycerols (i.e., asymmetric triacylglycerols composed of L which represents a long-chain saturated fatty acid having from 16 to 22 carbon atoms and U which represents an unsaturated fatty acid having from 16 to 22 carbon atoms) and glyceryl ether lipids wherein a long-chain alkyl or alkenyl chain is attached to the 1- or 3-position of the glycerin via an ether bond.

2. The lipase inhibitor according to claim 1, wherein the selected fat-soluble substance as the active ingredient is an SLS type triacylglycerol (i.e., symmetric triacylglycerol composed of S which represents a short-chain fatty acid having from 2 to 6 carbon atoms and L represents a long-chain fatty acid having from 16 to 22 carbon atoms).

3. A lipid absorption inhibitor containing the SLS type triacylglycerol described in claim 2 as the active ingredient.

4. An anti-obesity agent containing the SLS type triacylglycerol described in claim 2 as the active ingredient.

5. A hyperlipemia ameliorating agent containing the SLS type triacylglycerol described in claim 2 as the active ingredient.

6. A food product containing the agent according to any one of claims 2 to 5.

7. A pharmaceutical composition containing the agent according to any one of claims 2 to 5.

8. The lipase inhibitor according to clam 1, wherein the selected fat-soluble substance as the active ingredient is LUU type and UUL type triacylglycerols (i.e., asymmetric triacylglycerols composed of L which represents a long-chain saturated fatty acid having from 16 to 22 carbon atoms and U which represents an unsaturated fatty acid having from 16 to 22 carbon atoms).

9. A lipid absorption inhibitor containing the asymmetric triacylglycerol described in claim 8 as the active ingredient.

10. An anti-obesity agent containing the asymmetric triacylglycerol described in claim 8 as the active ingredient.

11. A hyperlipemia ameliorating agent containing the asymmetric triacylglycerol described in claim 8 as the active ingredient.

12. A food product containing the agent according to any one of claims 8 to 11.

13. A pharmaceutical composition containing the agent according to any one of claims 8 to 11.

14. The lipase inhibitor according to claim 1, wherein the selected fat-soluble substance as the active ingredient is a glyceryl ether lipid in which a long-chain alkyl or alkenyl chain is attached to the 1- position or the 3-position of the glycerin via an ether bond.

15. A lipid absorption inhibitor containing the glyceryl ether lipid described in claim 14 as the active ingredient.

16. An anti-obesity agent containing the glyceryl ether lipid described in claim 14 as the active ingredient.

17. A hyperlipemia ameliorating agent containing the glyceryl ether lipid described in claim 14 as the active ingredient.

18. A food product containing the agent according to any one of claims 14 to 17.

19. A pharmaceutical composition containing the agent according to any one of claims 14 to 17.
